Europäisches Patentamt

**European Patent Office**

**Office européen des brevets**

(11) Veröffentlichungsnummer: **0 227 757**
**B1**

(12) **EUROPÄISCHE PATENTSCHRIFT**

(45) Veröffentlichungstag der Patentschrifft:
07.02.90

(51) Int. Cl. 5: **A 61 F   9/00**

(21) Anmeldenummer: **86904081.6**

(22) Anmeldetag: **30.06.86**

(86) Internationale Anmeldenummer:
**PCT/DE 86/00268**

(87) Internationale Veröffentlichungsnummer:
**WO 87/00038 (15.01.87 Gazette 87/01)**

(54) **VORRICHTUNG ZUR LASERCHIRURGIE UND INSBESONDERE ZUR KERATOTOMIE DER CORNEA (III).**

(30) Priorität: **29.06.85 DE 3523341**

(43) Veröffentlichungstag der Anmeldung:
**08.07.87 Patentblatt 87/28**

(45) Bekanntmachung des Hinweises auf die Patenterteilung:
**07.02.90 Patentblatt 90/06**

(84) Bennante Vertragsstaaten:
**AT BE CH DE FR GB IT LI LU NL SE**

(56) Entgegenhaltungen:
**EP-A-0 089 921**
**DE-A-1 541 165**
**DE-A-2 202 120**
**US-A-394 713**
**US-A-2 009 816**

**Laser und Optoelektronik, volume 15, No 3, September 1983, Stuttgart (DE), E. SCHRÖDER et al.: Laser in der Augenheilkunde", pages 209-212**

**Nerem Record 1965, 04 November 1965, Engineering Meeting, C.J. KOESTER et al., "Design and Performance of a Laser Photocoagulator", page 154**

(73) Patentinhaber: **AESCULAP AG**
**Möhringer Strasse 125**
**D-7200 Tuttlingen (DE)**

(72) Erfinder: **SCHRÖDER, Eckhardt**
**Hans-Sachsstr. 9**
**D-8501 Eckental (DE)**

(74) Vertreter: **Hoeger, Stellrecht & Partner**
**Uhlandstrasse 14 c**
**D-7000 Stuttgart 1 (DE)**

LIBERGRAF, STOCKHOLM 1990

## Beschreibung

### Technisches Gebiet

Die Erfindung bezieht sich auf eine Vorrichtung zur Laserchirurgie und insbesondere zur Keratotomie der Cornea mit einem Laser, vorzugsweise einem UV-Laser, gemäß dem Oberbegriff des Patentanspruchs 1.

### Stand der Technik

Beispielsweise aus dem Artikel "Excimer-Laser surgery of the Cornea" von Stephen L. Trokel (American Journal of Ophthalmology, 1983, Band 96, Seite 710 - 715) ist es bekannt, daß sich insbesondere Argon-Fluorid-Excimer-Laser mit einer Wellenlänge von 193 nm dazu eignen, Operationen an der Cornea auszuführen. Ziel derartiger Operationen ist es beispielsweise, durch Aufbringen eines bestimmten "Schnittmusters" anomale Krümmungen der Cornea zu beheben.

Ferner ist aus der wissenschaftlichen Literatur bekannt, daß sich auch Licht anderer Wellenlänge zur Keratotomie der Cornea eignet.

Bei den bekannten Vorrichtungen zur Keratotomie der Cornea, wie sie beispielsweise in dem vorstehend genannten Artikel beschrieben und bei der Formulierung des Oberbegriffs des Patentanspruchs 1 vorausgesetzt sind, wird eine Blende dicht vor dem zu operierenden Auge angeordnet und mit UV-Laserlicht bestrahlt.

Bedingt durch diesen Aufbau haben die bekannten Vorrichtungen gemäß dem Oberbegriff des Patentanspruch 1 eine Reihe von Nachteilen:

Der Laserstrahl muß die Blende, in der sich das gewünschte "Schnittmuster" befindet, flächig beleuchten. Da das Schnittmuster nur einen kleinen Teil der beleuchteten Fläche einnimmt, steht für die Operation nur ein geringer Teil der Laserleistung tatsächlich zur Verfügung.

### Darstellung der Erfindung

Der Erfindung liegt die Aufgabe zugrunde, eine Vorrichtung zur Laserchirurgie und insbesondere zur Operation der Cornea anzugeben, bei der ein großer Teil der Laserleistung tatsächlich für die Operation zur Verfügung steht.

Eine erfindungsgemäße Lösung dieser Aufgabe ist mit ihren Weiterbildungen in den Patentansprüchen angegeben.

Erfindungsgemäß wird anstelle der Maske, in der das vollständige gewünschte Schnittmuster eingebracht ist, ein einzelner Spalt verwendet, dessen Ausrichtung bezüglich des Laserstrahls während des gesamten Operationsvorgangs unverändert bleibt, und der lediglich bezüglich seiner Länge und seiner Breite geändert wird. Im Anschluß an den Spalt ist eine optische Drehvorrichtung vorgesehen, die die Lage des Spaltbildes auf dem zu operierenden Gewebe, beispielsweise der Cornea dreht. Da der Laserstrahl nur noch den Spalt zu beleuchten hat, dessen Winkelausrichtung ungeändert bleibt, und bei dem sich lediglich die Länge und Breite in gewissen Grenzen ändern, steht für die Operation praktisch die gesamte Laserleistung zur Verfügung. Zwar sind bei einem komplizierten Schnittmuster die einzelnen Bestandteile des Schnittmusters, beispielsweise bei einem sternförmigen Schnittmuster die einzelnen Strahlen des Stern, nacheinander zu belichten, da aber bei jedem Belichtungsvorgang eine größere Strahlenleistung zur Verfügung steht, ergibt sich insgesamt eine kürzere Operationszeit als bei den bekannten Vorrichtungen, bei denen das gesamte Schnittmuster mit großen Absorptions- bzw. Reflexionsverlusten in der Blende beleuchtet wird.

Die optische Drehvorrichtung kann gemäß Anspruch 2 ein bekanntes Drehprisma, wie ein Dove-Prisma oder ein Oxley-Prisma sein. Besonders vorteilhaft ist es jedoch, das in Anspruch 3 beanspruchte Drehprisma zu verwenden, da bei diesem Prisma sämtliche Strahl-Übertrittsflächen senkrecht zum Strahlengang verlaufen und keine Grenzflächen etc. das Spaltbild unterbrechen.

Da das zu operierende Gewebe insbesondere bei der Operation der Cornea nicht eben, sondern typischerweise stark gekrümmt ist, ist es besonders vorteilhaft, die erfindungsgemäße Dreheinrichtung in Verbindung mit einer optischen Einrichtung zu verwenden, die das Spaltbild mit hoher Schärfentiefe auf das zu operierende Gewebe abbildet. Überraschenderweise hat es sich herausgestellt, daß eine derartige Einrichtung eine einfache telezentrische Anordnung zweier positiver optischer Elemente sein kann, zwischen denen der Spalt angeordnet ist, der auf das zu operierende Gewebe projiziert werden soll. Die optische Dreheinrichtung kann ebenfalls zwischen den beiden sammelnden optischen Elementen, die eine telezentrische Anordnung bilden, oder im Anschluß an die beiden Elemente angeordnet werden. Die Anordnung zwischen den beiden positiven optischen Elementen, hat den Vorteil, daß sich eine kurze und kompakte Bauweise ergibt.

Die erfindungsgemäße Vorrichtung nach einem der Patentansprüche kann jederzeit in ein Augen-optisches Untersuchungsgerät, wie ein Ophthalmoskop oder ein Spaltlampengerät integriert werden. Bei der Integration in ein Spaltlampengerät kann der Einbau beispielsweise in die Spaltleuchte selbst oder in den Trägerarm des Mikroskopes erfolgen, wenn der Laserstrahl durch den Trägerarm des Mikroskops auf das Auge projiziert werden soll. Eine derartige Vorrichtung ist bereits für einen Neodym-YAG-Laser vorgeschlagen worden, während Vorrichtungen, bei denen die Einspiegelung durch die Spaltleuchte erfolgt, bereits für Argon-Laser vorgeschlagen worden sind. Diese bekannte Strahlführung kann auch für Vorrichtungen mit Excimer-Lasern oder Lasern anderer Wellenlänge, wie HF-Lasern übernommen werden, wobei es besonders vorteilhaft ist, sämtliche Umlenkeinrichtungen als Quarzglas-Prismen auszuführen, da bei diesen die Reflexionsverluste wesentlich geringer als bei vergüteten Spiegelflächen sind.

Darüberhinaus ist es auch möglich, die erfindungsgemäße Vorrichtung nach einem der Patentansprüche in eine Vorrichtung zu integrieren, wie sie in der am gleichen Tage eingereichten Patentanmeldung WO-A-87/00036 beschrieben worden ist: Bei dieser Vorrichtung wird die Spaltleuchte nicht geschwenkt, sondern lediglich linear verschoben. Zusätzlich ist auch die Kinnstütze beweglich ausgeführt. Bei dieser Vorrichtung ist die Zahl der Spiegelflächen, die für die Einspiegelung des Laserstrahls benötigt werden, besonders gering, so daß ein besonders großer Teil der Laserleistung für die Operation zur Verfügung steht. Die Reduzierung der zur Strahlführung verwendeten Spiegel gleicht dabei eventuelle Verluste aufgrund der Drehprismen aus.

In jedem Fall hat die erfindungsgemäße Vorrichtung nach einem der Patentansprüche, den Vorteil, daß ein wesentlich größerer Teil der Laserleistung als beim Stand der Technik für die Operation zur Verfügung steht. Darüberhinaus kommt der erfindungsgemäß vorgesehenen Beleuchtung eines bezüglich seiner Ausrichtung ortsfesten Spaltes die Tatsache entgegen, daß ein Excimer-Laser ohne strahlformende Maßnahmen bereits einen länglichen Querschnitt hat, so daß eine Strahl-Querschnittsform, die den Spalt deckend beleuchtet, mit einfachen Maßnahmen, beispielsweise mit einer Telezenter-Anordnung oder mit Zylinderlinsen erreicht werden kann.

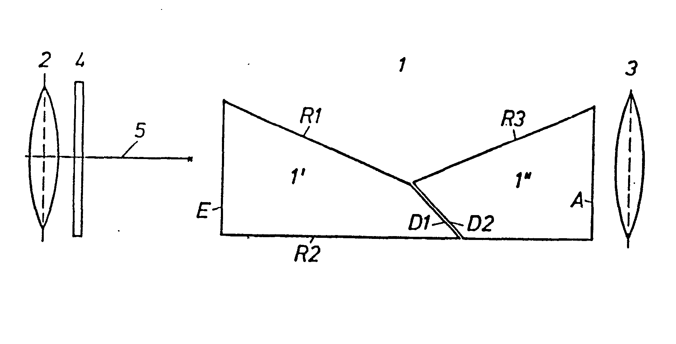

## Kurze Beschreibung der Zeichnung

Die Erfindung wird nachstehend anhand der Zeichnung exemplarisch beschrieben, deren einzige Figur einen Querschnitt durch eine erfindungsgemäße Vorrichtung zeigt.

## Weg zur Ausführung der Erfindung

In der Figur ist ein Drehprisma 1 dargestellt, das zwischen zwei optischen Elementen 2 und 3 mit positiver Brechkraft angeordnet ist. Zwischen dem optischen Element 2 und dem Drehprisma 1 befindet sich ein Spalt 4, dessen Winkel-Ausrichtung zu dem Laserstrahl 5 eines nicht dargestellten Excimer-Lasers unverändert bleibt, und dessen Länge und Breite entsprechend den jeweiligen Erfordernissen einstellbar sind.

Die Linsen 2 und 3 bilden eine telezentrische Anordnung, d.h. die Summe ihrer Brennweiten ist gleich ihrem (optischen) Abstand. Durch die telezentrische Anordnung der beiden positiven optischen Elemente 2 und 3 wird in Verbindung mit der Kohärenz des Laserstrahls 5 erreicht, daß der Spalt 4 mit großer Schärfentiefe auf das zu operierende Gewebe abgebildet wird. Beispielsweise erhält man bei einer Brennweite des optischen Elements 2 von ca. 200 mm und einer Brennweite des optischen Elements 3 von ca. 50

– 100 mm eine Schärfentiefe von mehreren Millimetern, so daß das Spaltbild konturscharf auch auf den krümmsten Teil der Cornea abgebildet wird.

Ausdrücklich wird darauf hingewiesen, daß diese hohe Schärfentiefe sich durch die Kohärenz des Laserlichts in Verbindung mit der telezentrischen Anordnung der beiden positiven optischen Elemente 2 und 3 ergibt. Bei Beleuchtung des Spaltes mit inkohärentem Licht, beispielsweise dem Licht einer Spaltlampe beim Ausrichten des Spaltbildes erhält man keine Abbildung mit so hoher Schärfentiefe wie bei Beleuchtung mit kohärentem Licht.

Die optische Dreheinrichtung 1 besteht aus zwei Teilen 1' und 1". Der Teil 1' weist eine senkrecht zum Laserstrahl 5 stehende Eintrittfläche E, Reflexionsflächen R1 und R2 sowie eine Strahlübertrittsfläche D auf, durch die der Strahl in den Teil 1" des Prismas übertritt. Die Reflexionsfläche R1 ist zum Laserstrahl 5 vor dem Prisma, d.h. zur optischen Achse um einen Winkel von 22,5° in der der Zeichnung entnehmbaren Richtung geneigt, während die Reflexionsfläche R2 parallel zur optischen Achse verläuft. Die Durchtrittsfläche D1 schließt mit der optischen Achse einen 45°-Winkel in der der Zeichnung entnehmbaren Richtung ein.

Der Teil 1" des Drehprismas weist eine Strahleintrittsfläche D2, eine Reflexionsfläche R3 und eine Strahlaustrittsfläche A auf, die auf der optischen Achse senkrecht steht. Die Strahleintrittsfläche D2 verläuft parallel zur Fläche D1, während die Reflexionsfläche R3 einen 22,5°-Winkel in der der Zeichnung entnehmbaren Richtung mit der optischen Achse 5 einschließt.

Zwischen den beiden Flächen D1 und D2 befindet sich ein Luftspalt. Alternativ hierzu sind die beiden Flächen aneinander angesprengt, jedoch nicht verkittet.

Das gezeigt Prisma hat eine Reihe von Vorteilen:

Gegenüber bekannten Oxley-Prismen weist die Reflexionsfläche R2 keine Unterbrechung durch eine Kittfläche etc. auf, so daß sich im Spaltbild keine Störungen ergeben. Trotzdem ist das erfindungsgemäß vorgeschlagene Prisma leicht herzustellen.

Vorstehend ist die erfindungsgemäße Vorrichtung exemplarisch beschrieben worden. Im Rahmen des allgemeinen Erfindungsgedankens, einen bezüglich seiner Ausrichtung feststehenden Spalt zu verwenden, dessen Bild auf dem zu operierenden Gewebe mittels einer optischen Dreheinrichtung gedreht wird, sind die verschiedensten Modifikationen möglich.

Natürlich kann die optische Dreheinrichtung immer dann, wenn es auf hohe Konturschärfe nicht ankommt, oder wenn das zu operierende Gewebe nicht extrem gekrümmt ist, auch ohne eine Projektionseinrichtung verwendet werden, die eine hohe Schärfentiefe des Spaltbildes ergibt.

Bei Verwendung der vorstehend beschriebenen Projektionseinrichtung für hohe Schärfentiefe, die aus zwei sammelnden optischen Elementen besteht, kann die optische Dreheinrichtung auch nach dem zweiten sammelnden Element angeordnet werden.

In der Zeichnung sind schematisch die optischen Elemente 2 und 3 als einfache Linsen dargestellt. Natürlich können diese optischen Elemente auch aus mehreren Linsen bestehen und gegebenenfalls zusätzlich so ausgelegt sein, daß sie den Strahlquerschnitt des Laserstrahls so beeinflussen, daß er den Spalt ohne größere Randverluste "deckend" ausleuchtet.

Darüberhinaus können auch besondere Maßnahmen vorgesehen werden, die den von vornherein bereits ovalen Querschnitt eines Excimer-Lasers so aufweiten, daß die Verluste durch den Spalt-Randbereich möglichst gering sind.

Vor allem ist es möglich, zusätzlich eine Ziel- und Ausrichteinrichtung vorzusehen, die Licht im sichtbaren Bereich auf dem gleichen Lichtweg wie der UV-Laser durch die erfindungsgemäße Vorrichtung projiziert, so daß die Lage des Spaltbildes auf dem zu operierenden Gewebe optisch kontrolliert werden kann. Diese optische Zieleinrichtung kann beispielsweise dadurch realisiert werden, daß die erfindungsgemäße Vorrichtung in eine Spaltleuchte eingebaut wird. Darüberhinaus ist es ferner natürlich auch möglich, einen zweiten Laser, beispielsweise einen HeliumNeon-Laser vorzusehen, der im sichtbaren Bereich arbeitet.

In jedem Falle sollten dann die optischen Elemente 2 und 3 als UV-Achromate ausgeführt werden: Beispielsweise können sie dann aus $CaF_2$ und $SiO_2$ bestehen.

Vor allem aber ist es möglich, die erfindungsgemäße Vorrichtung, die auf dem Grundgedanken basiert, den von vornherein ovalen Strahl eines UV-Excimer-Lasers zur Beleuchtung eines bezüglich seiner Winkel-Ausrichtung feststehenden Spaltes zu verwenden und das Spaltbild auf dem zu operierenden Gewebe optisch zu drehen, in beliebige optische und insbesondere augenoptische Untersuchungsgeräte, wie Ophthalmoskope, Spaltlampengeräte etc. zu integrieren.

Ferner ist es natürlich auch möglich, andere Laser zu verwenden, beispielsweise HF-Laser mit einer Wellenlänge von etwa 3 μm.

## Patentansprüche

1. Vorrichtung zur Laserchirurgie und insbesondere zur Keratotomie der Cornea, mit einem Laser, insbesondere einem UV-Laser, dessen Strahl auf das zu operierende Gewebe abbildbar ist und in dessen Strahlengang eine Blende angeordnet ist, die den beleuchteten Bereich des Gewebes begrenzt, dadurch *gekennzeichnet*, daß die Blende ein bezüglich seiner Winkel-Ausrichtung feststehender Spalt (4) ist, dessen Länge und Breite verstellbar sind, und daß zur Drehung des Spaltbildes zwischen dem Spalt (4) und dem zu operierenden Gewebe ein optisches Drehelement (1) angeordnet ist, das den Strahl um die optische Achse (5) rotiert.

2. Vorrichtung nach Anspruch 1, dadurch *gekennzeichnet*, daß das optische Drehelement (1) ein an sich bekanntes Drehprisma, wie ein Dove-Prisma oder ein Oxley-Prisma ist.

3. Vorrichtung nach Anspruch 1, dadurch *gekennzeichnet*, daß das Drehelement (1) ein aus zwei Teilen (1', 1") bestehendes Prisma ist, dessen Eintritts-(E) und Austrittsfläche (A) senkrecht auf der optischen Achse (5) stehen, und das drei Flächen (R1, R2, R3), die den Strahl total reflektieren und deren Flächennormalen mit der optischen Achse einen Winkel von 67,5°, 90° und 112,5° einschließen, sowie zwei Durchtrittsflächen (D1, D2) zwischen den beiden Teilen aufweist, durch die der Strahl senkrecht hindurchtritt.

4. Vorrichtung nach einem der Ansprüche 1 bis 3, dadurch *gekennzeichnet*, daß der Spalt (4) zwischen zwei sammelnden optischen Elementen (2, 3) angeordnet ist, die eine telezentrische Anordnung bilden.

5. Vorrichtung nach Anspruch 4, dadurch *gekennzeichnet*, daß die optische Dreheinrichtung (1) ebenfalls zwischen den sammelnden optischen Elementen (2, 3) angeordnet ist.

6. Vorrichtung nach einem der Ansprüche 1 bis 5, dadurch *gekennzeichnet*, daß zur Ausrichtung des Spaltbildes auf dem zu operierenden Gewebe eine Zielstrahleinrichtung vorgesehen ist.

7. Vorrichtung nach Anspruch 6, dadurch *gekennzeichnet*, daß die Zielstrahleinrichtung eine Spaltleuchte ist, deren Spalt gleichzeitig der Spalt (4) für einen Laser, der Licht im nicht sichtbaren Wellenlängenbereich erzeugt, beispielsweise einen UV-Laser, ist.

## Claims

1. A device for laser surgery and in particular for keratomy of the cornea, having a laser, in particular a UV laser, whose beam may be imaged onto the tissue to be operated and in whose beam path there is arranged a diaphragm which limits the illuminated tissue area, characterized in that the diaphragm is a slit (4) fixed with regard to its angle orientation and adjustable in length and width, and in that there is arranged an optical rotating element (1) for rotating the slit image between the slit (4) and the tissue to be operated, this rotating element rotating the beam about the optical axis (5).

2. A device in accordance with Claim 1, characterized in that the optical rotating element (1) is a rotating prism which is known per se. e.g. a Dove prism or an Oxley prism.

3. A device in accordance with Claim 1, characterized in that the rotating element (1) is a prism which is composed of two parts (1', 1"), whose entrance- (E) and emergence- (A) surfaces are perpendicular to the optical axis (5), which has three surfaces (R1, R2, R3) which totally reflect the beam and whose surface normals with the optical axis form an angle of 67.5°, 90° and 122.5°, and has two through-passage surfaces (D1, D2) which are situated between the two parts and through which the beam vertically passes.

4. A device in accordance with any one of Claims 1 to 3, characterized in that the slit (4) is arranged between two converging optical elements (2, 3) which form a telecentric arrangement.

5. A device in accordance with Claim 4, characterized in that the optical rotating device (1) is also arranged between the converging, optical elements (2, 3).

6. A device in accordance with any one of Claims 1 to 5, characterized in that there is a beam targeting device for orientating the slit image on the tissue to be operated.

7. A device in accordance with Claim 6, characterized in that the beam targeting device is a split lamp whose split is simultaneously the split (4) for a laser, for example a UV laser, which produces light in the invisible wavelength range.

**Revendications**

1. Dispositif pour la chirurgie au laser et en particulier pour la kératotomie de la cornée, comportant un laser, en particulier un laser UV, dont le faisceau peut donner une image sur le tissu à opérer et sur le chemin du faisceau duquel est disposé un diaphragme qui limite la zone éclairée du tissu, *caractérisé* en ce que le diaphragme est une fente (4) d'orientation angulaire fixe et dont la longueur et la largeur sont réglables et en ce que, pour faire tourner l'image de la fente, est disposé, entre la fente (4) et le tissu à opérer, un élément à rotation optique (1) qui fait tourner le faisceau autour de l'axe optique (5).

2. Dispositif selon la revendication 1, *caractérisé* en ce que l'élément à rotation optique (1) est un prisme à rotation connu en soi, comme un prisme Dove ou un prisme Oxley.

3. Dispositif selon la revendication 1, *caractérisé* en ce que l'élément à rotation (1) est un prisme constitué de deux parties (1', 1") dont la surface d'entrée (E) et la surface de sortie (A) sont perpendiculaires à l'axe optique (5), et qui présente trois surfaces (R1, R2, R3) qui réfléchissent totalement le faisceau et dont les normales à la surfaces font avec l'axe optique un angle de 67,5°, 90° et 112,5°, ainsi que, entre les deux parties, deux surfaces de passage (D1, D2) à travers lesquelles le faisceau passe perpendiculairement.

4. Dispositif selon l'une des revendications 1 à 3, *caractérisé* en ce que la fente (4) est disposée entre deux éléments optiques convergents (2, 3) qui forment une disposition télécentrique.

5. Dispositif selon la revendication 4, *caractérisé* en ce que le dispositif optique à rotation (1) est également disposé entre les éléments optiques convergents (2, 3).

6. Dispositif selon l'une des revendications 1 à 5, *caractérisé* en ce qu'un mécanisme de visée est prévu pour orienter l'image de la fente sur le tissu à opérer.

7. Dispositif selon la revendication 6, *caractérisé* en ce que le mécanisme de visée est un dispositif d'éclairage à collimateur dont la fente est simultanément la fente (4) d'un laser qui produit la lumière dans le domaine des longueurs d'onde non visibles, par exemple d'un laser UV.